# EUROPÄISCHE PATENTSCHRIFT

(11) **EP 1 242 172 B1**
(45) Veröffentlichungstag und Bekanntmachung des Hinweises auf die Patenterteilung: **15.10.2003**
(21) Anmeldenummer: 00983277.5
(22) Anmeldetag: 11.12.2000
(51) Int. Cl.: B01F 17/00, A61K 7/00, A61K 7/48

(54) **VERWENDUNG VON NANOSKALIGEN POLYMEREN**
USE OF NANOSCALE POLYMERS
UTILISATION DE POLYMERES NANOMETRIQUES

(30) Priorität: 18.12.1999 DE 19961277
(43) Veröffentlichungstag der Anmeldung: 25.09.2002
(73) Patentinhaber: Cognis Deutschland GmbH & Co. KG, 40589 Düsseldorf (DE)
(72) Erfinder: HENSEN, Hermann, 42781 Haan (DE); EGGERS, Anke, 40215 Düsseldorf (DE); SEIPEL, Werner, 40723 Hilden (DE)
(86) Internationale Anmeldenummer: EP0012518
(87) Internationale Veröffentlichungsnummer: WO01043859

(56) Entgegenhaltungen:
- EP-A- 0 240 424
- EP-A- 0 659 929
- WO-A-93/00076
- WO-A-99/43427

## Beschreibung

Die Erfindung befindet sich auf dem Gebiet der Nanopartikel und betrifft die Verwendung von nanoskaligen Polymeren in oberflächenaktiven kosmetischen Zubereitungen.

### Stand der Technik

Kosmetische Zubereitungen, welche Polymere enthalten, werden sowohl in Reinigungs- und Pflegemittein für Haut und Haar eingesetzt. Ihrer Anwendung führt nicht nur zu einer Verbesserung der Sensorik, der Kämmbarkeit der Haare sondem die statische Aufladung wird ebenfalls vermindert. Die Wirkung dieser Polymeren hängt jedoch stets mit der Verteilbarkeit und der Geschwindigkeit zusammen, mit der die Verbindungen eingebaut, adsorbiert bzw. resorbiert werden. Hier besteht für die verfügbaren Stoffe des Stands der Technik noch ein erhebliches Verbesserungspotential.

Die Aufgabe der Erfindung hat somit darin bestanden, die Aufnahme von Polymeren bei ihrer Applikation durch Bereitstellung neuer Anbietungsformen zu beschleunigen. Weiterhin sollen sie nach der Anwendung eine lang anhaltende Wirkung zeigen, eine gute dermatologische Verträglichkeit aufweisen und sich durch eine hervorragende Stabilität bei Temperaturiagerung auszeichnen.

Die Verwendung von nanoskaligen Polymeren in Medikamenten und der Textilpflege ist z.B. aus den Dokumenten WO-A-9300076, EP-A-0240424 and EP-A-0659929 bekannt.

### Beschreibung der Erfindung

Gegenstand der Erfindung ist die Verwendung von nanoskaligen Polymeren mit einem mittleren Durchmesser im Bereich von 10 bis 500 nm zur Herstellung von oberflächenaktiven kosmetischen Zubereitungen.

Überraschenderweise wurde gefunden, daß sowohl die Stabilität von Zubereitungen, wie beispielsweise Lotionen, Cremes und Körperreinigungspräparaten, als auch deren Konsistenz und konditionierende, pflegende Wirkung durch den Zusatz von Polymeren signifikant verbessert wird, wenn diese in Form von Nanoteilchen, d.h. Partikeln mit einem mittleren Durchmesser im Bereich von 10 bis 500, vorzugsweise 50 bis 300 und insbesondere 100 bis 150 nm vorliegen. Gleichzeitig zeigen derartige Zubereitungen gute dermatologische Verträglichkeiten und verbesserte sensorische Eigenschaften an Haut und Haar sowie eine Steigerung des Haarglanzes und insbesondere des Haarvolumens. Zusätzlich bewirken diese Verbindungen auf Keratinfaser einen deutlichen Antisplisseffekt, d.h. sie verbessern die Kämmbarkeit und reduzieren die statische Aufladung zwischen den Fasern.

### Polymere

Als Polymere kommen anionische, zwitterionische, amphotere und nichtionische Verbindungen in Frage, wie beispielsweise Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und deren Ester, unvemetzte und mit Polyolen vemetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxyproyl-methacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte natürliche Polymere, wie beispielsweise Guar, Cellulose, Stärke und/oder Alquinate, Proteine und Proteinderivate und Silicone in Frage.

### Herstellung von Nanopartikeln

Ein Verfahren zur Herstellung von Nanoteilchen durch rasche Entspannung von überkritischen Lösungen (**Rapid Expansion of Supercritical Solutions RESS**) ist beispielsweise aus dem Aufsatz von S.Chihlar, M.Türk und K.Schaber in **Proceedings World Congress on Particle Technology 3, Brighton, 1998** bekannt In einer bevorzugten Ausrührungsform der Erfindung setzt man nanoskalige Polymeren ein, die man erhält, indem man
(a) die Ausgangsstoffe unter überkritischen oder nahekritischen Bedingungen in einem geeigneten Lösungsmittel löst,
(b) die fluide Mischung über eine Düse in ein Vakuum, ein Gas oder eine Flüssigkeit entspannt, und
(c) das Lösemittel dabei gleichzeitig verdampft.

Um zu verhindern, daß die Nanoteilchen wieder zusammenbacken, empfiehlt es sich, die Ausgangsstoffe in Gegenwart geeigneter Schutzkolloide oder Emulgatoren zu lösen und/oder die kritischen Lösungen in wäßrige und/oder alkoholische Lösungen der Schutzkolloide bzw. Emulgatoren oder aber in kosmetische Öle zu entspannen, welche ihrerseits wieder gelöste Emulgatoren und/oder Schutzkolloide enthalten können. Geeignete Schutzkolloide sind dabei z.B. Gelatine, Casein, Gummi arabicum, Lysalbinsäure, Stärke sowie Polymere, wie etwa Polyvinylalkohole, Polyvinylpyrrolidone Polyalkylenglycole und Polyacrylate. Die bevorzugt zu verwendenden nanoskaligen Polymeren sind also die, die von einem Schutzkolloid und/oder einem Emulgator ummantelt vorliegen. Üblicherweise werden die Schutzkolloide oder Emulgatoren in Mengen von 0,1 bis 20, vorzugsweise 5 bis 15 Gew.-% - bezogen auf die Polymeren - eingesetzt.

Ein weiteres geeignetes Verfahren zur Herstellung der nanoskaligen Teilchen bietet die Evaporationstechnik. Hierbei werden die Ausgangsstoffe zunächst in einem geeigneten organischen Lösungsmittel (z.B. Alkane, pflanzliche Öle, Ether, Ester, Ketone, Acetale und dergleichen) gelöst. Anschließend werden die Lösungen derart in Wasser oder einem anderen Nicht-Lösungsmittel, gegebenenfalls in Gegenwart einer darin gelösten oberflächenaktiven Verbindung gegeben, daß es durch die Homogenisierung der beiden nicht miteinander mischbaren Lösungsmittel zu einer Ausfällung der Nanoteilchen kommt, wobei das organische Lösungsmittel vorzugsweise verdampft Anstelle einer wäßrigen Lösung können auch O/W-Emulsionen bzw. O/W-Mikroemulsionen eingesetzt werden. Als oberflächenaktive Verbindungen können die bereits eingangs erläuterten Emulgatoren und Schutzkolloide verwendet werden. Eine weitere Möglichkeit zur Herstellung von Nanoteilchen besteht in dem sogenannten GAS-Verfahren (Gas Anti Solvent Recrystallizadon). Das Verfahren nutzt ein hochkomprimiertes Gas oder überkritisches Fluid (z.B. Kohlendioxid) als Nicht-Lösungsmittel zur Kristallisation von gelösten Stoffen. Die verdichtete Gasphase wird in die Primärlösung der Ausgangsstoffe eingeleitet und dort absorbiert, wodurch sich das Flüssigkeitsvolumen vergrößert, die Löslichkeit abnimmt und feinteilige Partikel ausgeschieden werden. Ähnlich geeignet ist das PCA-Verfahren (Precipitation with a Compressed Fluid Anti-Solvent). Hier wird die Primärlösung der Ausgangsstoffe in ein überkritisches Fluid eingeleitet, wobei sich feinstverteilte Tröpfchen bilden, in denen Diffusionsvorgänge ablaufen, so daß eine Ausfällung feinster Partikel erfolgt. Beim PGSS-Verfahren (Particles from Gas Saturated Solutions) werden die Ausgangsstofte durch Aufpressen von Gas (z.B. Kohlendioxid oder Propan) aufgeschmolzen. Druck und Temperatur erreichen nahe- oder überkritische Bedingungen. Die Gasphase löst sich im Feststoff und bewirkt eine Absenkung der Schmelztemperatur, der Viskosität und der Oberflächenspannung. Bei der Expansion durch eine Düse kommt es durch Abkühlungseffekte zur Bildung feinster Teilchen.

### Gewerbliche Anwendbarkeit

Gegenüber Polymeren des Stands der Technik bewirkt die besondere Feinteiligkeit der Partiket eine verbesserte Konditionierwirkung, eine erhöhte Stabilität und Konsistenz der Emulsionen. Die nanoskaligen Polymere können zur Herstellung von kosmetischen Zubereitungen, wie beispielsweise Emulsionen, Cremes, Gele und Lotionen zur Hautpflege wie auch Shampoos, Duschbädern, Spülungen, Conditionem, Antisplissmitteln und dergleichen für die Haarpflege verwendet werden. Die Einsatzmenge der Polymeren liegt dabei üblicherweise in der Größenordnung von 0,1 bis 10, vorzugsweise 0,2 bis 5 und insbesondere 0,3 bis 2 Gew.-% - bezogen auf die Zubereitungen.

Die oberflächenaktiven Zubereitungen können femer als weitere Hilfs- und Zusatzstoffe milde Tenside, Ölkörper, Emulgatoren, Überfettungsmittel, Periglanzwachse, Konsistenzgeber, Verdickungsmittel, Siliconverbindungen, Fette, Wachse, biogene Wirkstoffe, Deodorantien, Filmbildner, Quellmittel, Antioxidantien, Hydrotrope, Konservierungsmittel, Solubilisatoren, Parfümöle, Farbstoffe und dergleichen enthalten.

Typische Beispiele für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetate und/oder Proteinfettsäurekondensate, letztere vorzugsweise auf Basis von Weizenproteinen.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, O-leyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestem mit Polyolen, Siliconöle und/oder aliphatische bzw. naphthenische Kohlenwasserstoffe, wie z.B. wie Squalan, Squalen oder Dialkylcyclohexane in Betracht.

Als **Emulgatoren** kommen beispielsweise nichtionogene Tenside aus mindestens einer der folgenden Gruppen in Frage:
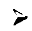 Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
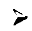 Alkyl- und/oder Alkenyloligoglykoside mit 8 bis 22 Kohlenstoffatomen im Alk(en)ylrest und deren ethoxylierte Analoga;
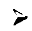 Anlagerungsprodukte von 1 bis 15 Mol Ethylenoxid an Ricinusöl und/oder gehärtetes Ricinusöl;
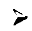 Anlagerungsprodukte von 15 bis 60 Mol Ethylenoxid an Ricinusöl und/oder gehärtetes Ricinusöl;
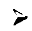 Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
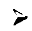 Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
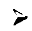 Mischester aus Pentaerythrit, Fettsäuren, Citronensäure und Fettalkohol gemäß DE 1165574 PS und/oder Mischester von Fettsäuren mit 6 bis 22 Kohlenstoffatomen, Methylglucose und Polyolen, vorzugsweise Glycerin oder Polyglycerin.
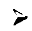 Mono-, Di- und Trialkylphosphate sowie Mono-, Di- und/oder Tri-PEG-alkylphosphate und deren Salze;
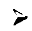 Wollwachsalkohole;
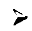 Polysiloxan-Polyalkyl-Polyether-Copolymere bzw. entsprechende Derivate;
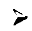 Polyalkylenglycole sowie
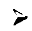 Glycerincarbonat.

Die **Antagerungsprodukte von Ethylenoxid undloder von Propylenoxid** an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C₁₂/₁₈-Fettsäuremono- und -diester von Anlagerungsprodukten von Ethylenoxid an Glycerin sind aus **DE 2024051 PS** als Rückfettungsmittel für kosmetische Zubereitungen bekannt.

**Alkyl- und/oder Alkenyloligoglycoside**, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für geeignete **Partialglyceride** sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als **Sorbitanester** kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete **Polyglycerinester** sind Polyglyceryl-2 Dipolyhydroxystearate (Dehymuls® PGPH), Polyglycerin-3-Diisostearate (Lameform® TGI), Polyglyceryl-4 Isostearate (Isolan® Gl 34), Potyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische.
Beispiele für weitere geeignete **Polyolester** sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen. Weiterhin können als Emulgatoren **zwitterionische Tenside** verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglydnat, N-AcyJam»nopropyJ-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöfmungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten.

Geeignete **Verdickungsmittel** sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, femer höhermolekulare Polyethylenglycolmono- und -diester von Fettsäuren, Polyacrylate, (z.B. Carbopole® von Goodrich oder Synthalene® von Sigma), Polyacrylamide, Polyvinytalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinytacetat/Butyimaleat/lsobomylacrylat-Copolymere, Methylvinylether/Maleinsäureanhydrid-Copolymere und deren Ester, unvemetzte und mit Polyolen vemetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxyproylmethacrylat-Copolymere, Polyvinylpyrrolidon, VinylpyrrolidonNinylacetat-Copolymere, Vinylpyrrolidon/DimethylaminoethylmethacrylaWinylcaprolactam-Terpolymere sowie gegebenenfalls derivatisierte Celluloseether und Silicone in Frage

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. 91, 27 (1976)**.

Typische Beispiele für **Fette** sind Glyceride, als **Wachse** kommen u.a. natürliche Wachse, wie z.B. Candelillawachs, Camaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage.
Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

Kosmetische **Deodorantien** (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker sowie Antitranspirantien fungieren.

Als **keimhemmende Mittel** sind grundsätzlich alle gegen grampositive Bakterien wirksamen Stoffe geeignet, wie Z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Famesol, Phenoxyethanol, Glycerinmonolaurat (GML), Diglycerinmonocaprinat (DMC), Salicylsäure-N-alkylamide wie z. B. Salicylsäure-n-octylamid oder Salicylsäure-n-decylamid.

Als **Enzyminhibitoren** sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Cognis GmbH, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder-phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

Als **Geruchsabsorber** eignen sich Stoffe, die geruchsbildende Verbindungen aufnehmen und weitgehend festhalten können. Sie senken den Partialdruck der einzelnen Komponenten und verringem so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcydohexylacetat, Linalylacetat, Phenylethylacetat, Linalylbenzoat, Benzylformiat, Allylcyclohexylpropionat, Styrallylpropionat und Benzylsalicylat. Zu den Ethem zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

**Antitranspirantien** (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
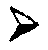 adstringierende Wirkstoffe,
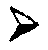 Ölkomponenten,
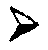 nichtionische Emulgatoren,
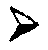 Coemulgatoren,
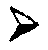 Konsistenzgeber,
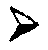 Hilfsstoffe wie z. B. Verdicker oder Komplexierungsmittel und/oder
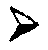 nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
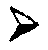 entzündungshemmende, hautschützende oder wohlriechende ätherische Öle,
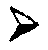 synthetische hautschützende Wirkstoffe und/oder
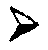 öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil. 108, 95 (1993) entnommen werden.

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
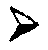 Glycerin;
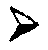 Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
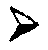 technische Oligoglyceringemische mit einem Eigenkondensationsgrad von 1,5 bis 10 wie etwa technische Diglyceringemische mit einem Diglyceringehalt von 40 bis 50 Gew.-%;
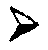 Methyolverbindungen, wie insbesondere Trimethylolethan, Trimethylolpropan, Trimethylolbutan, Pentaerythrit und Dipentaerythrit;
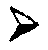 Niedrigalkylglucoside, insbesondere solche mit 1 bis 8 Kohlenstoffen im Alkylrest, wie beispielsweise Methyl- und Butylglucosid;
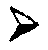 Zuckeralkohole mit 5 bis 12 Kohlenstoffatomen, wie beispielsweise Sorbit oder Mannit,
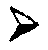 Zucker mit 5 bis 12 Kohlenstoffatomen, wie beispielsweise Glucose oder Saccharose;
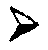 Aminozucker, wie beispielsweise Glucamin;
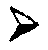 Dialkoholamine, wie Diethanolamin oder 2-Amino-1,3-propandiol.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutem und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethem zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, a-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Beispiele

Zur Herstellung der nanoskaligen Polymere **(Beispiele 1 bis 6)** wurde zunächst Kohlendioxid einem Reservoir mit einem konstanten Druck von 60 bar entnommen und über eine Kolonne mit einer Aktivkohle- und einer Molekularsieb-Packung gereinigt. Nach der Verflüssigung wurde das CO₂ mit Hilfe einer Diaphragma-Pumpe bei einer konstanten Fördermenge von 3,5 l/h auf den gewünschten überkritischen Druck p verdichtet. Anschließend wurde das Lösungsmittel in einem Vorheizer auf die erforderliche Temperatur T1 gebracht und in eine Extraktionskolonne (Stahl, 400 ml) geleitet, welche mit den Wachsen beladen war. Die resultierende überkritische, d.h. fluide Mischung wurde über eine lasergezogene Düse (Länge 830 µm, Durchmesser 45 µm) bei einer Temperatur T2 in eine Plexiglas Expansionskammer versprüht, die eine 4 Gew.-%ige wäßrige Lösung eines Emulgators bzw. Schutzkolloids enthielt. Das fluide Medium verdampfte und zurück blieben die im Schutzkolloid eingeschlossenen, dispergierten Nanopartikel. Die Verfahrensbedingungen und der mittlere Partikelgrößenbereich (photometrisch nach der 3-WEM-Methode bestimmt) sind in der nachfolgenden Tabelle 1 angegeben.

**Tabelle 1**

| **Nanopartikel** | | | | | | | |
|---|---|---|---|---|---|---|---|
| **Bsp.** | **Polymer** | **Lsgm** | **p bar** | **T1 °C** | **T2 °C** | **Emulgator/Schutzkolloid** | **PGB nm** |
| 1 | Polymer IR 400 | CO₂ | 200 | 80 | 175 | Polyvinylalkohol | 60-120 |
| 2 | Coschedia Guar C 261 N | CO₂ | 180 | 70 | 160 | Polyethylenglycol (M=400) | 75-120 |
| 3 | Merquat Guar C 261 | CO₂ | 200 | 85 | 180 | Polyvinylalkohol | 75-130 |
| 4 | Luviquat Care | CO₂ | 200 | 85 | 175 | Polyvinylalkohol | 60-140 |
| 5 | Luviskol VAG 4W | CO₂ | 200 | 85 | 175 | Polyvenylalkohol | 55-140 |
| 6 | Silicon Risin | CO₂ | 200 | 85 | 175 | Polyvinylalkohol | 60-140 |
| 7 | Carbopol EDT 2020 | CO₂ | 200 | 85 | 175 | Polyvinylalkohol | 60-140 |

Zur Untersuchung der Haaravivage wurden Haarsträhnen vor der Nullmessung mediumblondiert. Die Trockenkämmbarkeit wurde unter Zulassung der elektrostatischen Aufladung untersucht. nach einer Einwirkzeit von 5 min wurden die Testlösungen (1g/1g Haar) unter Standardbedingungen (38 °C, 11/min) 1 min gespült. Die Messung wurde an 20 Haarsträhnen durchgeführt. Eine ausführliche Beschreibung der Meßmethoden befindet sich in **J.Soc.Cosm.Chem., 24, 782 (1973)**. Die Ergebnisse sind in Tabelle 2 zusammengefaßt. Angegeben ist jeweils die Restarbeit bzw. Restaufladung bezogen auf den Ausgangswert. Die Beurteilung des Haarglanzes erfolgte auf einer Skala von 1 bis 5. Die Beispiele 1 bis 4 sind erfindungsgemäß, die Versuche V1 und V2 dienen zum Vergleich.

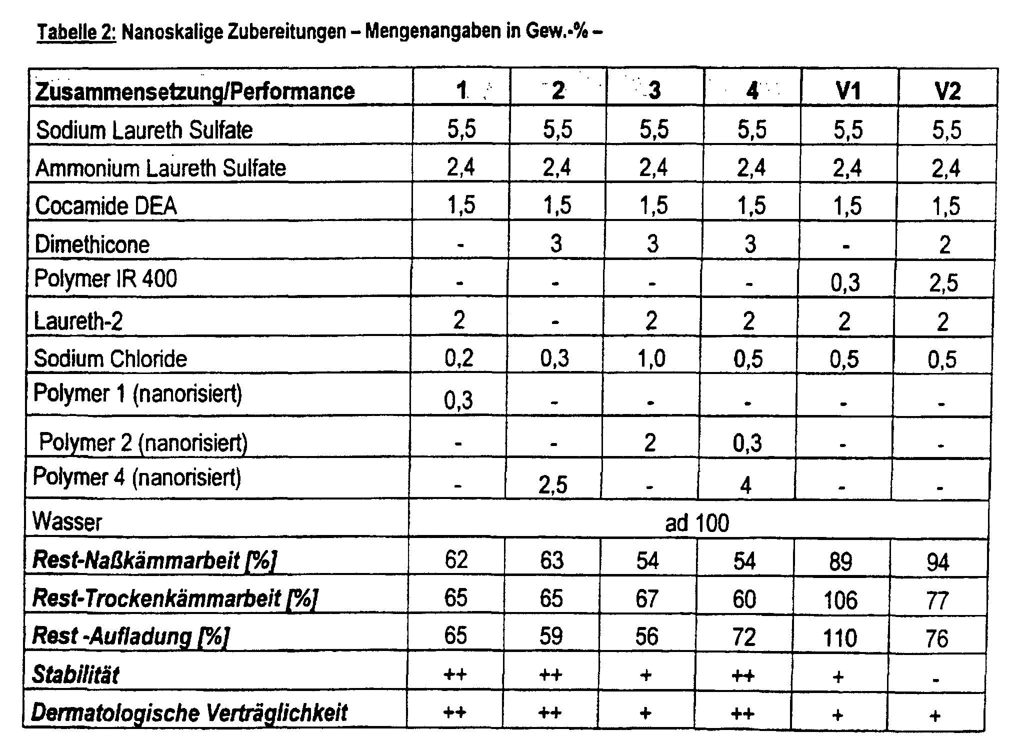

Die nachfolgende Tabelle 3 enthält eine Reihe von Formulierungsbeispielen mit kationischen Nanopartikeln.

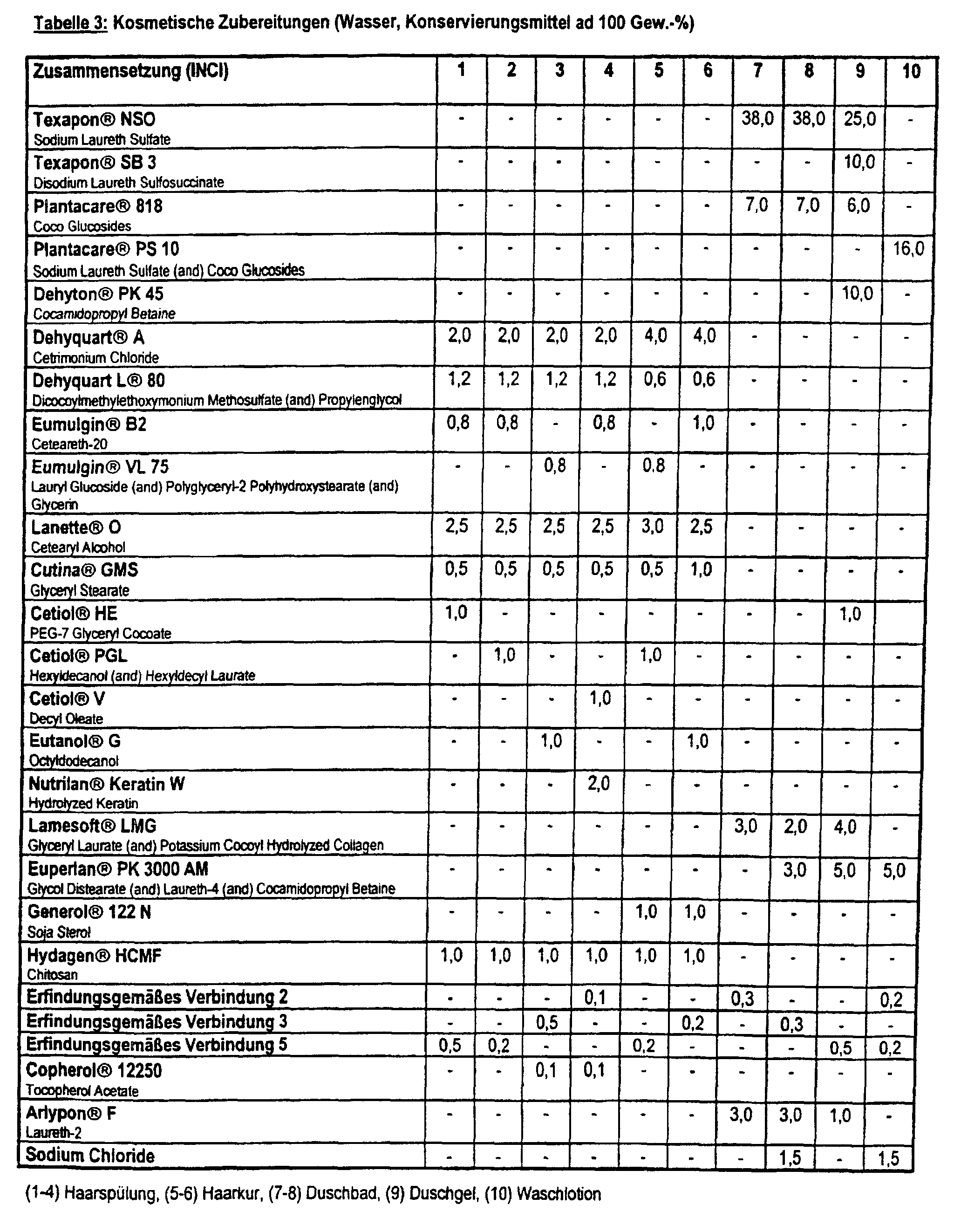

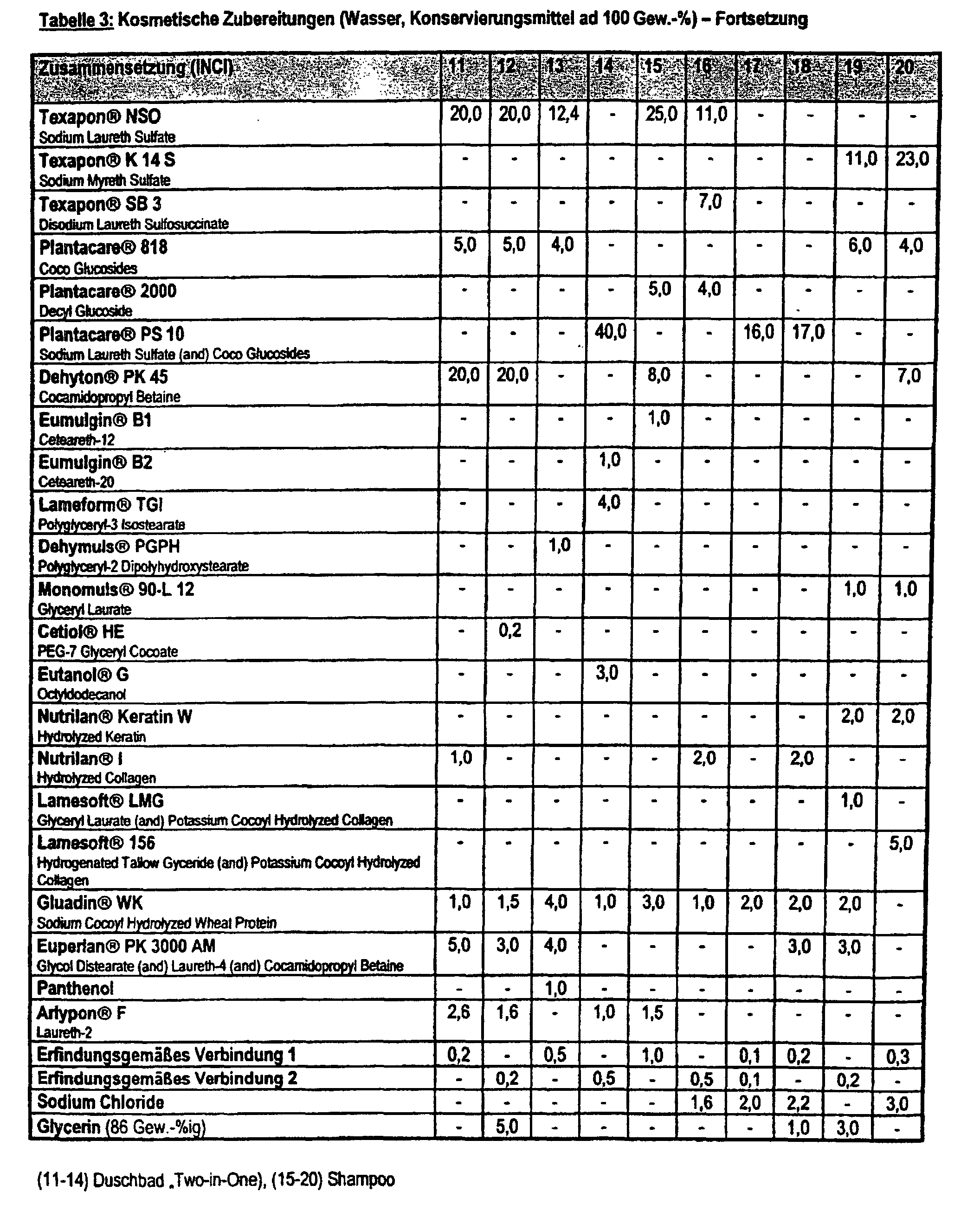

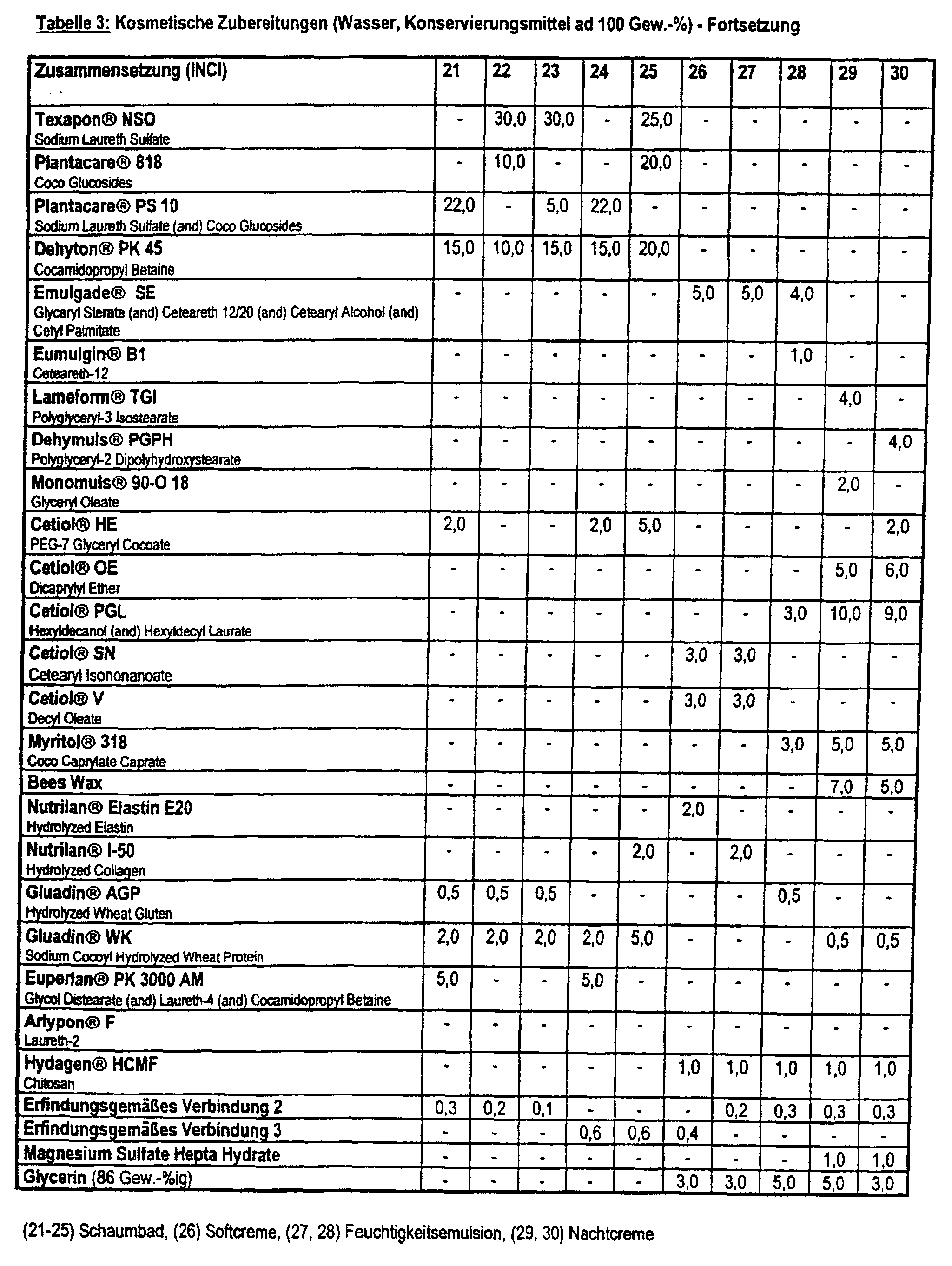

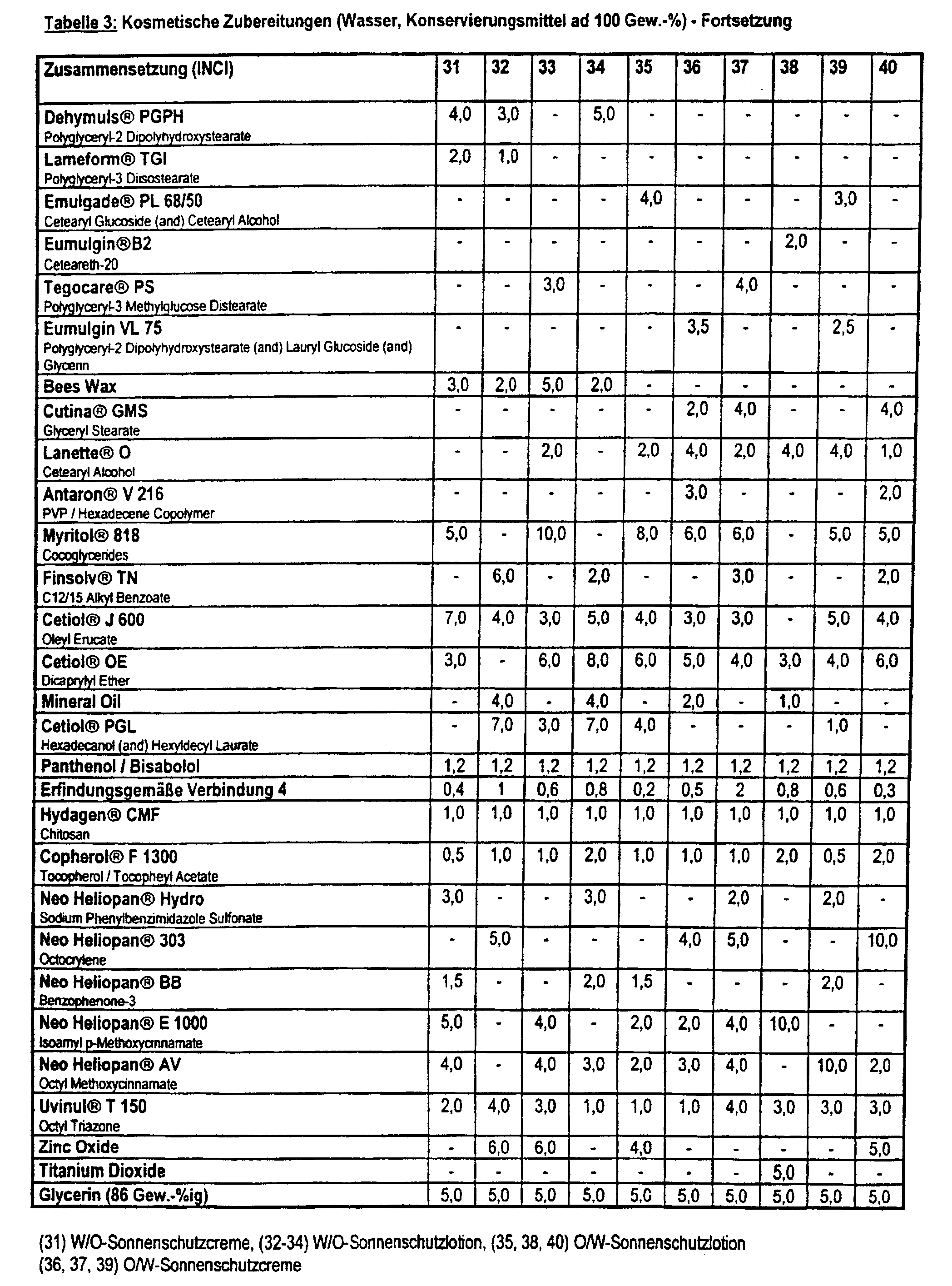

## Patentansprüche

1. Verwendung von nanoskaligen Polymeren in Form von Partikeln mit einem mittleren Durchmesser im Bereich von 10 bis 500 nm zur Herstellung von oberflächenaktiven kosmetischen Zubereitungen.

2. Verwendung nach Anspruch 1, **dadurch gekennzeichnet, daß** man anionische, zwitterionische, amphotere und nichtionische Polymere einsetzt.

3. Verwendung nach den Ansprüchen 1 und/oder 2, **dadurch gekennzeichnet, daß** man Polymere einsetzt, die ausgewählt sind aus der Gruppe, die gebildet wird von Vinylacetat/Crotonsäure-Copolymeren, VinylpyrrolidonNinylacrylat-Copolymeren, Vinylacetat/Butylmaleat/ Isobomylacrylat-Copolymeren, Methylvinylether/Maleinsäureanhydrid-Copolymeren und deren Ester, unvemetzten und mit Polyolen vemetzte Polyacrylsäuren, Acrylamidopropyltrimethylammoniumchlorid/ Acrylat-Copolymeren, Octylacrylamid/Methylmethacrylat/tert.Butytaminoethyl-methacrylat/2-Hydroxyproylmethacrylat-Copolymeren, Polyvinylpyrrolidon, Vinylpyrrolidon/Vinylacetat-Copolymeren, Vinylpyrrolidon/Dimethylaminoethylmethacrylat/Vinylcaprolactam-Terpolymeren sowie gegebenenfalls derivatisierten natürlichen Polymeren, Proteinen, Proteinderivaten und Siliconen.

4. Verwendung nach mindestens einem der Ansprüche 1 bis 3, **dadurch gekennzeichnet, daß** man Polymeren einsetzt, die man erhält, indem man
(a) die Ausgangsstoffe unter überkritischen oder nahekritischen Bedingungen in einem geeigneten Lösungsmittel löst,
(b) die fluide Mischung über eine Düse in ein Vakuum, ein Gas oder eine Flüssigkeit entspannt, und
(c) das Lösemittel dabei gleichzeitig verdampft.

5. Verwendung nach mindestens einem der Ansprüche 1 bis 4, **dadurch gekennzeichnet, daß** man Nanopartikel einsetzt, welche von einem Schutzkolloid ummantelt vorliegen.

6. Verwendung nach Anspruch 5, **dadurch gekennzeichnet, daß** man als Schutzkolloid Polyvinylalkohol oder Polyethylenglycol einsetzt.

7. Verwendung nach mindestens einem der Ansprüche 1 bis 6, **dadurch gekennzeichnet, daß** man die Polymeren in Mengen von 0,1 bis 10 Gew.-% - bezogen auf die Zubereitungen - einsetzt.

## Claims

1. The use of nanoscale polymers in the form of particles with a mean diameter of 10 to 500 nm for the production of surface-active cosmetic preparations.

2. The use claimed in claim 1, **characterized in that** anionic, zwitterionic, amphoteric and nonionic polymers are used.

3. The use claimed in claims 1 and/or 2, **characterized in that** polymers selected from the group consisting of vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers and optionally derivatized natural polymers such as, for examle, guar, cellulose, starch and/or alginates, proteins and protein derivatives and silicones are used.

4. The use claimed in at least one of claims 1 to 3, **characterized in that** polymers obtained by
(a) dissolving the starting materials in a suitable solvent under supercritical or near-critical conditions,
(b) expanding the fluid mixture through a nozzle into a vacuum, a gas or a liquid and
(c) simultaneously evaporating the solvent
are used.

5. The use claimed in at least one of claims 1 to 4, **characterized in that** nanoparticles coated with a protective colloid are used.

6. The use claimed in claim 5, **characterized in that** plyvinyl alcohol or polyethylene glycol is used as the protective colloid.

7. The use claimed in at least one of claims 1 to 6, **characterized in that** the polymers are used in quantities of 0.1 to 10% by weight, based on the preparations.

## Revendications

1. Utilisation de polymères à l'échelle du nanomètre sous forme de particules ayant un diamètre moyen dans la zone de 10 à 500 µm, en vue de la production de préparations cosmétiques actives sur la tension superficielle.

2. Utilisation selon la revendication 1,
**caractérisée en ce qu'**
on met en oeuvre des polymères anioniques, zwitterioniques, amphoètres, et non ioniques.

3. Utilisation selon les revendications 1 et/ou 2,
**caractérisée en ce qu'**
on met en oeuvre des polymères choisis dans le groupe formé des copolymères acétate de vinyle/acide crotonique, des copolymères acétate de vinyle/maléate de butyle/acrylate d'isobornyle, des copolymères éther méthylvinilique/acide maléique anhydre et de leurs esters, des acides polyacryliques non réticulés et réticulés avec des polyols, les copolymères chlorure d'acrylamidopropythriméthylammonium/acrylate, les copolymères octylacrylamide/méthacrylate de méthyle/méthacrylate de tertbutyle aminoéthyle/méthacrylate de 2-hydroxypropyle, la polyvinylpyrrolidone, les copolymères vinylpyrrolidone/acétate de vinyle, les terpolymères vinylpyrrolidone/méthacrylate de diméthylaminoéthyle/vinylcaprolactame ainsi que les polymères naturels éventuellement convertis, les protéines, les dérivés de protéines et les silicones.

4. Utilisation selon au moins une des revendications 1 à 3,
**caractérisée en ce qu'**
on met en oeuvre des polymères que l'on obtient selon un procédé dans lequel :
a) on dissout les matières de départ dans des conditions hypercritiques ou proches de la critique, dans un solvant approprié,
b) ont détend le mélange fluide par l'intermédiaire d'une buse dans un vide, un gaz ou un liquide et
c) on évapore le solvant en même temps.

5. Utilisation selon au moins une des revendications 1 à 4,
**caractérisée en ce qu'**
on met en oeuvre des nanoparticules que se présentent enrobées par un colloïde protecteur.

6. Utilisation selon la revendication 5,
**caractérisée en ce qu'**
on met en oeuvre comme colloïde protecteur de l'alcool polyvinylique ou du polyéthylèneglycol.

7. Utilisation selon au moins une des revendications 1 à 6,
**caractérisée en ce qu'**
on met en oeuvre les polymères en quantités allant de 0,1 à 10 % en poids rapportés aux préparations.
